# EUROPÄISCHE PATENTSCHRIFT

(11) **EP 1 572 613 B1**
(45) Veröffentlichungstag und Bekanntmachung des Hinweises auf die Patenterteilung: **16.08.2006**
(21) Anmeldenummer: 03785691.1
(22) Anmeldetag: 01.12.2003
(51) Int. Cl.: C07C 45/46, C07C 45/81, C07C 49/84, C07C 51/60, C07C 51/363

(54) **VERFAHREN ZUR HERSTELLUNG VON BENZOPHENONEN**
METHOD FOR THE PRODUCTION OF BENZOPHENONES
PROCEDE POUR PRODUIRE DES BENZOPHENONES

(30) Priorität: 13.12.2002 DE 10258669
(43) Veröffentlichungstag der Anmeldung: 14.09.2005
(73) Patentinhaber: BASF Aktiengesellschaft, 67056 Ludwigshafen (DE)
(72) Erfinder: MAYWALD, Volker, 67071 Ludwigshafen (DE); HOFFMANN, Nico, 67227 Frankenthal (DE); KEIL, Michael, 67251 Freinsheim (DE); VOGELBACHER, Uwe, Josef, 67071 Ludwigshafen (DE); WEVERS, Jan, Hendrik, 67591 Hohen-Suelzen (DE)
(86) Internationale Anmeldenummer: PCT/EP2003/013483
(87) Internationale Veröffentlichungsnummer: WO 2004/054953

(56) Entgegenhaltungen:
- EP-A- 0 897 904
- EP-A- 1 295 877
- WO-A-01/51440

## Beschreibung

Verfahren zur Herstellung von Benzophenonen der Formel I, 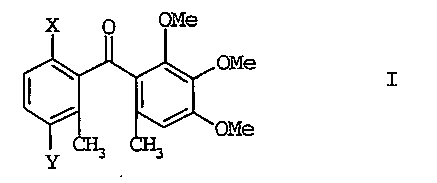 in der X Chlor, Hydroxy, Methoxy oder C₁-C₆-Alkylcarbonyloxy und Y Chlor oder Brom bedeuten können, durch Umsetzung eines Säurechlorids der Formel II, 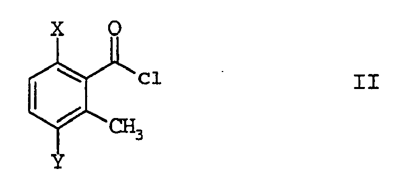 in der X und Y die obengenannte Bedeutung besitzen, mit 3,4,5-Trimethoxytoluol, dadurch gekennzeichnet dass die Reaktion in Gegenwart
a) eines aromatischen Kohlenwasserstoffe als Verdünnungsmittel und
b) 0,01 bis 0,2 mol% bezogen auf das Säurechlorid eines Eisenkatalysators
c) bei einer Temperatur, die zwischen 60°C und dem Siedepunkt des jeweiligen Verdünnungsmittels liegt, durchgeführt wird.

Die Benzophenone der Formel I sind aus EP-A 897 904 bekannt. Die Friedel-Crafts Acylierung wird hierbei mit stöchiometrischen Mengen Aluminiumchlorid oder Phosphorpentoxid durchgeführt. Als Verdünnungsmittel wurden Niedrigsieder wie Dichlormethan oder Benzol eingesetzt. Diese Verfahrensweise führt bei der technischen Realisierung zu zahlreichen Problemen. Insbesondere ist die wäßrige Aufarbeitung und der Zwangsanfall großer Mengen aluminium- bzw. phosphorhaltigen Abwassers von Nachteil.

In der WO 01/51440 wird ein Verfahren zur Herstellung der Benzophenone I beschrieben, das in Gegenwart von Eisen(III)chlorid und beträchtlichen Mengen Graphit arbeitet. Als Verdünnungsmittel wird 1,2-Dichlorethan eingesetzt. Die Ausbeute an isoliertem Benzophenon beträgt lediglich ca. 72%. Außerdem erfordert die Abtrennung des Graphits einen zusätzlichen Filtrierschritt.

Aufgabe der vorliegenden Erfindung war es daher, ein wirtschaftliches und selektives Verfahren zur Herstellung der Benzophenone I zu entwickeln, das mit katalytischen Mengen eines Friedel-Crafts Katalysators auskommt und gleichzeitig hohe Raum/Zeit-Ausbeuten liefert.

Überraschenderweise wurde nun gefunden, dass sich die im Stand der Technik vorhandenen Nachteile vermeiden lassen, wenn die Reaktion in Gegenwart
a) eines aromatischen Kohlenwasserstoffs und
b) 0,01 bis 0,2 mol% bezogen auf das Säurechlorid eines Eisenkatalysators
c) bei einer Temperatur, die zwischen 60°C und dem Siedepunkt des jeweiligen Verdünnungsmittels liegt, durchgeführt wird.

Als Eisenkatalysator werden in der Regel feingemahlenes Eisenpulver oder Eisen(III)salze verwendet. Bevorzugt ist Eisen(III)oxid und besonders bevorzugt Eisen (III) chlorid.

Als Verdünnungsmittel kommen die hochsiedenden und unter den Reaktionsbedingungen inerten aromatischen Kohlenwasserstoffe wie beispielsweise Chlorbenzol, Benzotrifluorid und Nitrobenzol in Frage. Besonders bevorzugt sind halogenierte aromatische Kohlenwasserstoffe und insbesondere Chlorbenzol.

Die Verwendung eines höhersiedenden Verdünnungsmittels hat weiterhin den Vorteil, dass die bei der Reaktion entstehende Salzsäure mittels eines Inertgasstromes, der vorzugsweise durch die Reaktionsmischung geleitet wird, entfernt werden kann, ohne dass nennenswerte Verluste des Verdünnungsmittels entstehen. Anhand der Herstellungsbeispiele wird ersichtlich, dass durch die Inertgasstrippung die Reaktionszeiten drastisch reduziert werden können. Somit lassen sich selbst bei sehr geringen Katalysatormengen, von beispielsweise kleiner 0,1 mol%, Reaktionszeiten von unter 10 Stunden realisieren, ohne dass größere Ausbeuteverluste in Kauf genommen werden müssen. Als Inertgas kommen Edelgase wie Argon, Luft und vorzugsweise Stickstoff in Frage. Der Inertgasstrom wird vorzugswiese durch die Reaktionsmischung geleitet. Vorteilhaft ist es beispielsweise eine möglichst feine Verteilung der Gaspartikel in der Reaktionsmischung zu erzielen. Dies kann beispielsweise mittels einer Düse oder eines Blasenrings erfolgen, wobei diese Mittel vorteilhaft unterhalb des Rührers angebracht sind. Die durch den Rektionsansatz geleitete Gasmenge richtet sich vor allem nach der Ansatzgröße. Pro mol Säurechlorid werden bis zu 5 1/h Inertgas eingeleitet.

Die Verwendung des erfindungsgemäßen Verdünnungsmittels hat weiterhin den Vorteil, dass die Friedel-Crafts Acylierung bei höheren Temperaturen durchgeführt werden kann, wodurch sich abermals die Reaktionszeiten reduzieren lassen. Im allgemeinen wird im Temperaturbereich von 60°C bis zum Siedepunkt des Verdünnungsmittels gearbeitet. Vorzugsweise im Temperaturbereich von 80 bis 150°C.

Der Eisenkatalysator wird in einem Molverhältnis von 0,01 bis 0,2 mol% bezogen auf das Säurechlorid eingesetzt. Vorzugsweise werden 0,03 bis 0,1 mol% des Katalysators verwendet.

Weiterhin wird das 3,4,5-Trimethoxytoluol in der Regel in Bezug auf das Säurechlorid in einem Molverhältnis von 1 bis 3 eingesetzt. Vorzugsweise wird ein leichter Überschuß von 1,05 bis 1,4 Moläquivalenten des 3,4,5-Trimethoxytoluols verwendet.

In einer bevorzugten Ausführungsform des Verfahrens wird das 3,4,5-Trimethoxytoluol ggf. im Verdünnungsmittel vorgelegt und der Eisenkatalysator und das Säurechlorid ggf. im Verdünnungsmittel innerhalb von 0,5 bis 20 Stunden, vorzugsweise 4 bis 6 Stunden abhängig von der gewählten Reaktionstemperatur zudosiert. Der Eisenkatalysator wird vorzugsweise im Säurechlorid gelöst zudosiert.

Die umgekehrte Fahrweise (Dosierung des 3,4,5-Trimethoxytoluols) hat als Eintopfvariante apparatetechnische Vorteile, wenn bereits das Säurechlorid im gleichen Reaktionsgefäß hergestellt wurde. Wie aus Tabelle 1 ersichtlich, führt diese Fahrweise zu einer etwas geringeren Selektivität und Ausbeute bei sonst gleicher Fahrweise.

In der Regel wird der Reaktionsansatz nach beendeter..Dosierung noch bis zu 20 Stunden und vorzugsweise 2 bis 4 Stunden nachgerührt. Die Nachrührzeit kann in der Regel verkürzt werden, wenn das Lösungsmittel und ggf. überschüssiges 3,4,5-Trimethoxytoluol am Ende der Friedel-Crafts Acylierung destillativ entfernt wird. Mit der Destillation kann bereits begonnen werden, wenn nur ein Teilumsatz erzielt wurde. Die Destillationszeit kann genutzt werden um den Umsatz zu vervollständigen.

Die destillative Abtrennung des Verdünnungsmittels ist die bevorzugte Aufarbeitungsvariante. Als Destillationssumpf verbleibt im Reaktionskessel eine Schmelze des gewünschten Benzophenons, die mit einem C₁-C₆-Alkohol vorzugsweise Methanol zur Kristallisation gebracht werden kann. Oft kann es von Vorteil sein, dem Alkohol geringe Mengen Wasser zuzusetzen, um die Eisensalze vollständig zu lösen.

Das erfindungsgemäße Verfahren eignet sich insbesondere zur Herstellung von 5-Bromo-2',6-dimethyl-2,4',5',6'-tetramethoxybenzophenon. Ferner lassen sich beispielsweise nach dem erfindungsgemäßen Verfahren: 2,5-Dichloro-2',6-dimethyl-4',5',6'-trimethoxybenzophenon, 5-Chloro-2',6-dimethyl-2-hydroxy-4',5',6'-trimethoxybenzophenon oder 5-Bromo-2',6-dimethyl-2-hydroxy-4',5',6'-trimethoxybenzophenon herstellen. Im Falle der beiden letztgenannten Verbindungen kann es ratsam sein, die freie Hydroxygruppe in 2-Stellung in Form einer C₁-C₆-Alkylcarbonyloxy-Gruppe zu schützen und nach Beendigung der Friedel-Crafts Acyliering wieder abzuspalten.

Das erfindungsgemäße Verfahren hat den Vorteil, dass ausschließlich die gewünschte trikline Modifikation anfällt. Bei den bisher bekannten Verfahren haben sich stets Gemische aus in der Regel zwei Modifikationen gebildet.

Die thermodynamisch stabilere, trikline Modifikation weist einen Schmelzpunkt von 99,5 bis 100,5°C auf und zeigt im IR-Spektrum charakteristische Banden bei 445, 568, 641, 769, 785 und 822 cm⁻¹.

Wie oben erwähnt liefern die Verfahren des Standes der Technik eine zweite, thermodynamisch instabilere Modifikation mit einem Schmelzpunkt von 91,5 bis 92,5°C und charakteristischen Banden bei 428, 648, 704 und 805 cm⁻¹ im IR-Spektrum.

Das erfindungsgemäße Verfahren hat weiterhin den Vorteil, daß die Herstellung des Säurechlorids II und auch die Bromierung zur Säure IIIa im gleichen Verdünnungsmittel wie die Friedel-Crafts Acylierung durchgeführt werden können. Wie in Schema 1 am Beispiel der Herstellung des

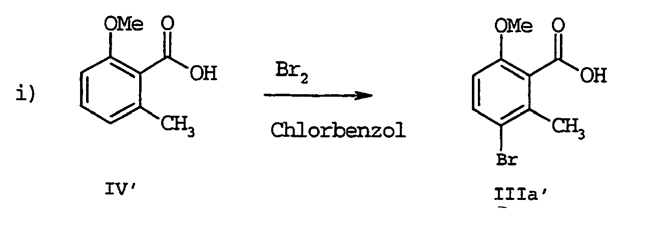

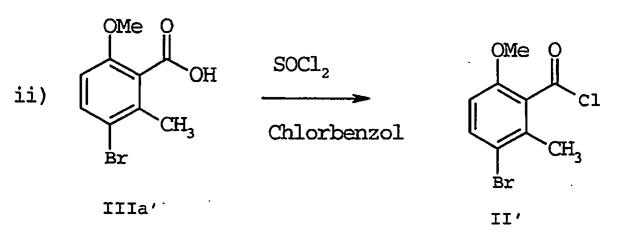

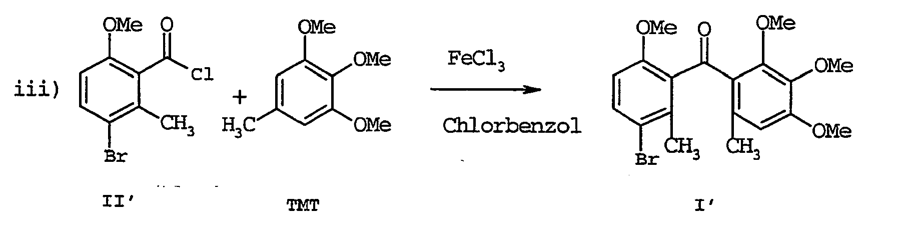

5-Bromo-2',6-dimethyl-2,4',5',6'-tetramethoxybenzophenons (I') gezeigt, lassen sich (i) die Bromierung der 2-Methoxy-6-methylbenzoesäure IV' zur 5-Brom-2-methoxy-6-methylbenzoesäure IIIa', (ii) die anschließende Umsetzung zum Säurechlorid II' und schließlich (iii) die Friedel-Crafts Acylierung zum Benzophenon I' allesamt in Chlorbenzol durchführen. Abhängig von der Dosierreihenfolge in der Friedel-Crafts Stufe können somit alle drei Reaktionsschritte in einer Eintopfvariante zusammengefasst werden.

Die höheren Siedepunkte des Verdünnungsmittels im erfindungsgemäßen Verfahren erlauben es weiterhin, die Einsatzstoffe Brom im Bromierungsschritt, Thionylchlorid (Phosgen) in der Säurechloridstufe und 3,4,5-Trimethoxytoluol in der Friedel-Crafts Stufe, die vorzugweise jeweils im Überschuß eingesetzt werden, destillativ abzutrennen und in den jeweiligen Prozeß (i bis iii) wieder rückzuführen. Werden halogenierte Kohlenwasserstoffe wie Benzotrichlorid oder Chlorbenzol als Verdünnungsmittel eingesetzt, so gelingt es in der Friedel-Crafts Stufe aufgrund der Siedepunktunterschiede (iii) destillativ ein Verdünnungsmittel zu erhalten, das frei von 3,4,5-Trimethoxytoluol ist und deshalb unmittelbar in die Bromierungsstufe (i) rückgeführt werden kann.

Die Bildung des Säurechlorids (Stufe ii) lässt sich wie in der Literatur im einzelnen beschrieben bewerkstelligen. Als Chlorierungsmittel werden in der Regel Thionylchlorid oder Phosgen eingesetzt. Die Reaktionstemperatur beträgt üblicherweise von Raumtemperatur bis 80°C.

Die Bromierung (Stufe i) kann wie in der Literatur beschrieben durchgeführt werden. Die Reaktion kann entweder in Gegenwart vorzugsweise jedoch ohne Säurekatalyse durchgeführt werden. Die Reaktionstemperatur beträgt im allgemeinen 0 bis 80°C.

### Verfahrensbeispiele

### Beispiele 1 bis 7

Allgemeine Verfahrensvorschrift zur Herstellung von 5-Bromo-2',6-dimethyl-2,4',5',6'-tetramethoxybenzophenons (I') ausgehend von 5-Brom-2-methoxy-6-methylbenzoesäurechlorid (II')

Eine Lösung von 1047 g (3,973 mol) 5-Brom-2-methoxy-6-methylbenzoesäurechlorid in 1715 g Chlorbenzol wurden mit 0,72 g (0,0044 mol) (Beispiele 1 bis 4und 7) bzw. 0,36 g (0,0022 mol) (Beispiel 5) bzw. 0,18 g (0,0011 mol) (Beipiel 6) wasserfreiem Eisen-(III)chlorid versetzt und zu einer Lösung von 868,7 g (4,768 mol) 3,4,5-Trimethoxytoluol in 467,8 g Chlorbenzol bei der in der Tabelle angegebenen Reaktionstemperatur während 4 h dosiert. Anschließend wurde das Reaktionsgemisch 2 h bei Reaktionstemperatur nachgerührt. Zur Entfernung der entstandenen HCl wurde während der Dosier- und Nachrührzeit ein konstanter Stickstoffstrom durch das Reaktionsgemisch geleitet (der jeweilige Volumenstrom kann der Tabelle entnommen werden). Anschließend wurde das Chlorbenzol bei 80 mbar und Temperaturen von 80-105°C abdestilliert. Reinheit und Ausbeute der rohen Produktschmelze wurden vor Kristallisation mittels quantitativer HPLC analysiert (Ergebnisse siehe Tabelle).

Zur Kristallisation des 5-Bromo-2',6-dimethyl-2,4',5',6'-tetramethoxybenzophenons (I') wurden 4900 g Methanol bei 50°C vorgelegt und die 105°C heisse Schmelze eingetragen. Die Kristallisation erfolgte durch Abkühlung mittels einer Temperaturrampe bis auf -5°C. Die Titelverbindung wurde durch Zentrifugation isoliert, auf der Zentrifuge mit Methanol gewaschen und getrocknet.

| Versuch | Molprozent FeCl₃ | N₂-Strom | Reaktions- temperatur | Umsatz nach 6 h | Selektiv ität | Ausbeute nach Dest. |
|---|---|---|---|---|---|---|
| 1 | 0,11 | 10 l/h | 80°C | 76,7 % | 99,3 % | 97,4 % |
| 2 | 0,11 | 10 l/h | 100°C | 90,4 % | 99,3 % | 97,5 % |
| 3 | 0,11 | 5 l/h | 120°C | 96,0 % | 98,9 % | 98,3 % |
| 4 | 0,11 | 10 l/h | 145°C | 100,0 % | 97,8 % | 97,5 % |
| 5 | 0,06 | 10 l/h | 145°C | 99,5 % | 99,4 % | 99,0 % |
| 6 | 0,03 | 10 l/h | 145°C | 99,3 % | 99,2 % | 98,9 % |
| | | | | | | |

| Vorlage des Säurechlorids | | | | | | |
|---|---|---|---|---|---|---|
| 7 | 0,11 | 10 l/h | 145°C | 100,0 % | 89,4 % | 89,4 % |

### Beispiel 8

Herstellung von 5-Bromo-2',6-dimethyl-2,4',5',6'-tetramethoxybenzophenons (I') ausgehend von 2-Methoxy-6-methylbenzoesäure (IV')

### (i) Herstellung von 5-Bromo-2-methoxy-6-methylbenzoesäure (IIIa')

700 g (4,212 mol) 2-Methoxy-6-methylbenzoesäure (IV') wurden in 2343,5 g Chlorbenzol suspendiert und anschließend 707,2 g (4,423 mol) elementares Brom bei einer konstanten Innentemperatur von 15°C innerhalb von 3 h zugetropft. Danach wurde 2 h bei 35°C gerührt. Anschließend wurden 628,7 g Chlorbenzol bei einer Innentemperatur von 77-82°C und 200 mbar abdestilliert, wobei das überschüssige Brom und HBr ebenfalls aus dem Reaktionsgefäß entfernt wurden. Das bromhaltige Chlorbenzoldestillat konnte nach Analyse des Bromgehalts ohne Ausschleusung im nächsten Ansatz wieder eingesetzt werden. Die dort einzusetzende Menge an Brom konnte entsprechend reduziert werden.

Die Zusammensetzung der Rohmischung wurde per quantitativem HPLC ermittelt. Erhalten wurden 980,5 g (4,0 mol = 95% Ausbeute) einer Suspension von IIIa' in Chlorbenzol. Die Selektivität der Bromierung ist hoch. Das Verhältnis von 5-Brom- zu 3-Bromverbindung liegt bei >500:1.

### (ii) Herstellung von 5-Bromo-2-methoxy-6-methylbenzoylchlorid (II')

Die unter (i) erhaltene Suspension wurde durch Zugabe von 754,8 g Chlorbenzol verdünnt und auf eine Temperatur von 50°C abgekühlt. Dann wurden 0,95 g (0,013 mol) Dimethylformamid zugegeben und anschließend 528,8 g (4,445 mol) Thionylchlorid bei einer Innentemperatur von 50°C innerhalb von 1,5 h zudosiert. Schließlich wurde bei 50°C weitere 1,5 h nachgerührt. Danach wurden 754,8 g Chlorbenzol bei einer Innentemperatur von 83-90°C bei 200 mbar abdestilliert, wobei auch überschüssiges Thionylchlorid und restliche Salzsäure und Schwefeldioxid aus dem Reaktionsgemisch entfernt wurden. Das Thionylchlorid-haltige Chlorbenzoldestillat konnte nach Analyse des Thionylchloridgehalts ohne Ausschleusung im nächsten Ansatz wieder verwendet werden. Die dort einzusetzende Menge an Thionylchlorid konnte entsprechend reduziert werden.

Der Wertgehalt des Destillationssumpfes wurde mittels quantitativer HPLC ermittelt. Erhalten wurden 1047 g (3,973 mol = 99,5% Ausbeute) 5-Brom-2-methoxy-6-methylbenzoylchlorid als Lösung in Chlorbenzol.

### iii) Herstellung von 5-Bromo-2',6-dimethyl-2,4',5',6'-tetramethoxybenzophenons (I')

Die Herstellung erfolgte anolog den Beispielen 1 bis 7 ebenfalls in Chlorbenzol. Hinsichtlich Ausbeute und Reinheit der gebildeten Produkte wurden vergleichbare Ergebnisse erzielt.

## Patentansprüche

1. Verfahren zur Herstellung von Benzophenonen der Formel I, 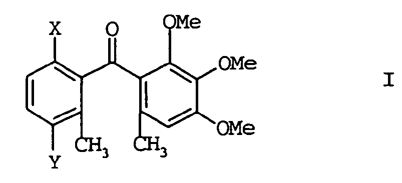 in der X Chlor, Hydroxy, Methoxy oder C₁-C₆-Alkylcarbonyloxy und Y Chlor oder Brom bedeuten können, durch Umsetzung von einem Säurechlorid der Formel II, 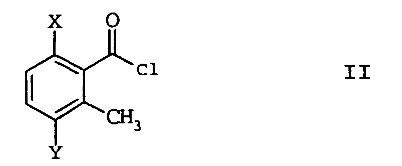 in der X und Y die obengenannte Bedeutung besitzen, mit 3,4,5-Trimethoxytoluol, **dadurch gekennzeichnet, dass** die Reaktion in Gegenwart
a) eines aromatischen Kohlenwasserstoffs ausgewählt aus der Gruppe: Chlorbenzol, Benzotrifluorid und Nitrobenzol als Verdünnungsmittel und
b) 0,01 bis 0,2 mol% bezogen auf das Säurechlorid eines Eisenkatalysators
c) bei einer Temperatur, die zwischen 60°C und dem Siedepunkt des jeweiligen Verdünnungsmittels liegt, durchgeführt wird.

2. Verfahren nach Anspruch 1, **dadurch gekennzeichnet, dass** als Verdünnungsmittel Chlorbenzol eingesetzt wird.

3. Verfahren nach den Ansprüchen 1 oder 2, **dadurch gekennzeichnet, dass** 3,4,5-Trimethoxytoluol ggf. im jeweiligen Verdünnungsmittel vorgelegt wird und das Säurechlorid einschließlich des Eisenkatalysators ggf. im jeweiligen Verdünnungsmittel zudosiert wird.

4. Verfahren nach den Ansprüchen 1 bis 3, **dadurch gekennzeichnet, dass** die bei der Reaktion entstehende Salzsäure durch Strippung mittels eines Inertgasstroms aus dem Reaktionsansatz entfernt wird.

5. Verfahren nach den Ansprüchen 1 bis 4, **dadurch gekennzeichnet, dass** das Verdünnungsmittel gegen Ende oder während des Verlaufs der Reaktion abdestilliert wird, und die verbleibende Produktschmelze in einem C₁-C₆-Alkohol zur Kristallisation gebracht wird.

6. Verfahren nach den Ansprüchen 1 bis 5, **dadurch gekennzeichnet, dass** das Säurechlorid der Formel II durch Umsetzung einer Säure der Formel III, 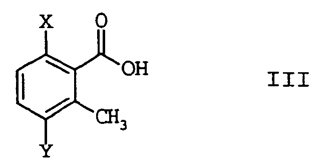 in der X und Y die obengenannte Bedeutung besitzen, mit Thionylchlorid oder Phosgen gegebenenfalls in Gegenwart von Dimethylformamid, im gleichen Verdünnungsmittel hergestellt wird, das auch in der folgenden Friedel-Crafts Stufe eingesetzt wird.

7. Verfahren nach Anspruch 6, **dadurch gekennzeichnet, dass** nach Bildung des Säurechlorids II zumindest ein Teil des Verdünnungsmittels mit überschüssigem Thionylchlorid abdestilliert und in den Prozess rückgeführt wird.

8. Verfahren nach Anspruch 6, **dadurch gekennzeichnet, dass** die Säure der Formel IIIa, 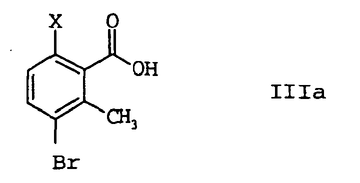 durch Bromierung der Verbindung IV, 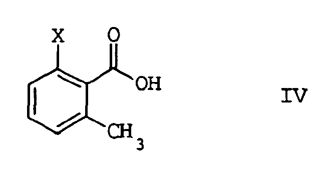 mit elementarem Brom im gleichen Verdünnungsmittel hergestellt wird, das auch in den folgenden beiden Stufen verwendet wird.

9. Verfahren nach Anspruch 8, **dadurch gekennzeichnet, dass** zumindest ein Teil des Verdünnungsmittels und überschüssiges Brom am Ende der Bromierung abdestilliert und in den Prozess rückgeführt wird.

## Claims

1. A process for preparing benzophenones of the formula I, 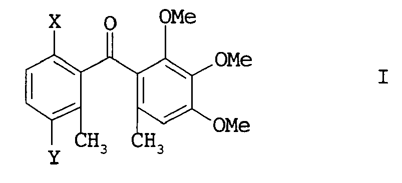 where X may be chlorine, hydroxyl, methoxy or C₁-C₆-alkylcarbonyloxy, and Y may be chlorine or bromine, by reacting an acid chloride of the formula II 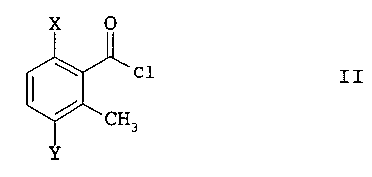 where X and Y are as defined above with 3,4,5-trimethoxytoluene, which comprises carrying out the reaction in the presence of
a) an aromatic hydrocarbon selected from the group of: chlorobenzene, benzotrifluoride and nitrobenzene as a diluent and
b) from 0.01 to 0.2 mol% of an iron catalyst, based on the acid chloride,
c) at a temperature between 60°C and the boiling point of the particular diluent.

2. The process according to claim 1, wherein the diluent used is chlorobenzene.

3. The process according to claim 1 or 2, wherein 3,4,5-trimethoxytoluene is initially charged, if appropriate in the particular diluent, and the acid chloride together with the iron catalyst is metered in, if appropriate in the particular diluent.

4. The process according to any of claims 1 to 3, wherein the hydrochloric acid forming in the reaction is removed from the reaction mixture by stripping using an inert gas stream.

5. The process according to any of claims 1 to 4, wherein the diluent is distilled off toward the end or during the course of the reaction, and the remaining product melt is crystallized in a C₁-C₆-alcohol.

6. The process according to any of claims 1 to 5, wherein the acid chloride of the formula II is prepared by reacting an acid of the formula III 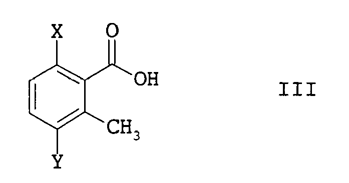 where X and Y are each as defined above with thionyl chloride or phosgene, if appropriate in the presence of dimethylformamide, in the same diluent which is also used in the subsequent Friedel-Crafts stage.

7. The process according to claim 6, wherein, after formation of the acid chloride II, at least a portion of the diluent is distilled off with excess thionyl chloride and recycled into the process.

8. The process according to claim 6, wherein the acid of the formula IIIa 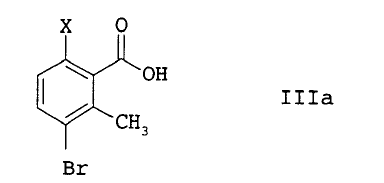 is prepared by brominating the compound IV 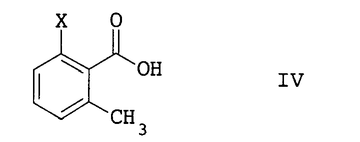 with elemental bromine in the same diluent which is also used in the two subsequent stages.

9. The process according to claim 8, wherein at least a portion of the diluent and excess bromine is distilled off at the end of the bromination and recycled into the process.

## Revendications

1. Procédé de préparation de benzophénones de la formule I 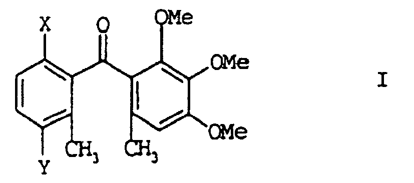 dans laquelle X peut représenter du chlore ou un groupe hydroxy, méthoxy ou C₁-C₆-alkylcarbonyloxy et Y du chlore ou du brome, par réaction d'un chlorure d'acide de la formule II 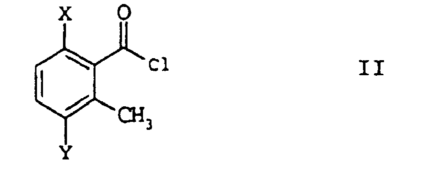 dans laquelle X et Y possèdent la signification précitée, avec du 3,4,5-triméthoxytoluène, **caractérisé en ce que** la réaction est effectuée en présence
a) d'un hydrocarbure aromatique choisi parmi le groupe : chlorobenzène, benzotrifluorure et nitrobenzène, comme diluant, et
b) de 0,01 à 0,2 % molaire, par rapport au chlorure d'acide, d'un catalyseur à base de fer,
c) à une température qui est située entre 60°C et le point d'ébullition du diluant respectif.

2. Procédé suivant la revendication 1, **caractérisé en ce que**, comme diluant, on met en oeuvre du chlorobenzène.

3. Procédé suivant la revendication 1 ou 2,
**caractérisé en ce qu'**on introduit préalablement du 3,4,5-triméthoxytoluène, éventuellement dans le diluant respectif, et on ajoute ensuite en dosant le chlorure d'acide, y compris le catalyseur à base de fer, éventuellement dans le diluant respectif.

4. Procédé suivant les revendications 1 à 3,
**caractérisé en ce que** l'acide chlorhydrique formé au cours de la réaction est éliminé de la masse réactionnelle par stripage au moyen d'un courant de gaz inerte.

5. Procédé suivant les revendications 1 à 4,
**caractérisé en ce que** le diluant est distillé vers la fin de la réaction ou pendant le déroulement de celle-ci et la masse fondue de produit subsistante est amenée à cristalliser dans un alcool en C₁-C₆.

6. Procédé suivant les revendications 1 à 5,
**caractérisé en ce que** le chlorure d'acide de la formule II est préparé par réaction d'un acide de la formule III 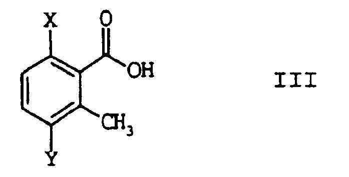 dans laquelle X et Y possèdent la signification précitée, avec du chlorure de thionyle ou du phosgène, éventuellement en présence de diméthylformamide, dans un diluant identique qui est aussi mis en oeuvre dans l'étape de Friedel-Crafts suivante.

7. Procédé suivant la revendication 6, **caractérisé en ce que**, après formation du chlorure d'acide II, au moins une partie du diluant est séparée par distillation avec du chlorure de thionyle excédentaire et est recyclée dans le processus.

8. Procédé suivant la revendication 6, **caractérisé en ce que** l'acide de la formule IIIa 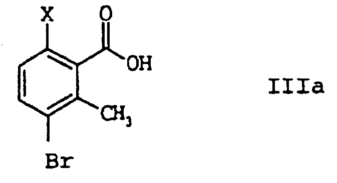 est préparé par bromation du composé IV 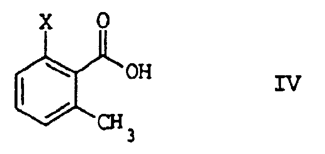 avec du brome élémentaire dans un diluant identique qui est aussi utilisé dans les deux étapes suivantes.

9. Procédé suivant la revendication 8, **caractérisé en ce qu'**au moins une partie du diluant et du brome excédentaire est séparée par distillation à la fin de la bromation et est recyclée dans le processus.
